# EUROPEAN PATENT APPLICATION

(11) **EP 1 969 997 A1**
(43) Date of publication of application: **17.09.2008**
(21) Application number: 07388013.0
(22) Date of filing: 12.03.2007
(51) Int. Cl.: A61B 5/00, G01N 21/03, G01N 21/35, G01N 33/49

(54) **Sensor system**

(71) Applicant: Radiometer Basel AG, 4051 Basel (CH)
(72) Inventor: Depeursinge, Christian, 1028 Preverenges (CH); Salzmann, Daniel, 1167 Lussy-sur-Morges (CH)
(74) Representative: Marnaes, Lene Meineche

(57) **Abstract**

A sensor system for detection of a gaseous chemical substance is provided, which comprises an optical sampling cell holding a sampling chamber of a volume of at most 20mm³, a light emitter and a light receiver. The sampling cell is adapted for free-space propagation of the light beam. With the sensor system, high sensitivity is obtained by elimination of interferometric noise.

## Description

The present invention relates to a sensor system and a sampling cell assembly for use with the sensor system.

Sensors for measuring parameters in a test fluid are widely used in various fields of chemistry, biology and physiology.

One such field is blood gas monitoring. The measurement of arterial blood gas parameters is an integral part of monitoring critical ill patients. Although analysis of arterial blood samples is considered to be the most accurate method for determining the blood gas status of a patient, the information provided by such punctual measurement reflects the situation only at the time a sample is taken. As blood gas values may change very rapidly in certain clinical conditions, in particular with patients having unstable respiratory or cardiopulmonary conditions, frequent or even continuous monitoring of blood gas parameters may be required to provide optimal care to the patient.
The need of such type of monitoring has led to the development of several invasive and non-invasive methods for the assessment of blood gas parameters. Among them, transcutaneous monitoring of blood gases is the only non-invasive technique available today permitting the simultaneous measurement of both oxygen and carbon dioxide partial pressures. The method has the unique feature of providing instant knowledge of the body's ability to deliver oxygen to the tissue and to remove carbon dioxide via the cardio-pulmonary system.

Preferably, sensors should have a high sensitivity, i.e. the ability of the sensor to detect and distinguish the true parameter signal from false signals. Sensor sensitivity is related to sample volume requirements and to the response time. At high sample volumes, sensitivity is likely to be high, however, so is the response time. At lower sample volumes, response time may be reduced, however, so is the sensor sensitivity. On the other hand, in many cases large sample volume may not be available at all.

In terms of small sample volumes, reference may be made to Pandraud, G. et al. in "Sensors and actuators B", Chemical 85 (2000) p. 158-162 where a sensor system for analysis of liquid samples is disclosed. The sensor system is based on evanescent wave sensing with an accent on optical absorption of the detected medium applying the Lambert-Beer law as read-out principle and micro-fluidic direct bonding for sampling cell construction and miniaturization. The resulting sample cell volume is about 0.8 mm³, well adapted for small volume analysis.

With many applications the analyte to be measured, however, may be in a gaseous phase, as is the case with blood gas monitoring. This, in turn, adds to the sensitivity requirements as the sample density for gaseous samples is low compared to liquid or solid samples.

In GB 2 219 656 a detection principle for analysis of gaseous samples is described which is a continuous signal (DC) detection using a collimated light beam and a retroreflector. Before returning back through the incoming optical fibre, the light beam interacts within the gas over twice the cell length defined as the distance between the collimator and the reflector. However, as the light is returned into the incoming optical fibre, the forward propagating mode will interfere with the back propagating mode and generate a resonator signature (interferometric noise) which will lower the system performance.

The breath gas analyzer described in US 6,599,253 is adapted for infrared spectroscopic monitoring the breath gas of a patient. The analyzer may detect gases in sample volumes in the order of 1000 mm³, corresponding to the requirements for human breath flow monitoring. For high frequent or continuous blood gas monitoring, however, the sampling volume is decades higher than the gas volume practically available, i.e. in the mm³-range.

In the particular field of transcutaneous blood gas sensing the requirements to the sensor system is to (1) access gas concentrations in the terms of gas permeation and diffusion within a short response time, (2) fulfil high sensitivity and high selectivity in a small volume, (3) access highly localized concentrations of blood gasses and (4) perform (1)-(3) in real-time monitoring.

With the introduction of MicroGas 7650 in 1993, a new generation of transcutaneous monitors was introduced. This sensor comprises the basic elements of a Clark-type *p*O₂ sensor and a Severinghaus-type *p*CO₂ sensor. *p*CO₂ is measured potentiometrically by determining the pH of an electrolyte. A change of pH is proportional to the logarithm of a *p*CO₂ change. The pH is determined by measuring the potential between a miniaturized pH glass electrode and an Ag/AgCl reference electrode.

However, despite the hitherto proposed sensor systems for small volume gas detection, e.g. gas in permeation, there is still a need for a sensor system which may combine high sensitivity, calibration-free operation, i.e. calibration during manufacture only, low sample volume and short response time. Accordingly, it is an object of the present invention to provide such a sensor system.

Thus, in one aspect of the invention, a sensor system for detection of a gaseous chemical substance in a medium is provided, which comprises
an optical sampling cell holding a sampling chamber for receiving a sample comprising the chemical substance, a light emitter for generating and coupling a light beam into the sampling cell for free-space propagation along a light beam optical path within the sampling cell for interaction with the sample held in the sampling chamber, and a light receiver to detect the light beam from the sampling cell and to produce an output signal reflecting the chemical substance of the sample, the sensor system being characterized in that the sampling chamber has a volume is at most 20 mm³.

Compared to prior art sensor systems, the present system requires only a very small sample volume of gas. Further, it provides a sensor system which is simpler and cheaper and has a higher sensitivity compared to the sensor technology described in e.g. WO03/023374 which is based on evanescent wave sensing.

The sensor system of the present invention provides drift-free and calibration-free operation as the optical measurement principle is based on the physical properties of the chemical substance detected. No chemical reactions are involved.

In the field of transcutaneous blood gas monitoring the sensor system allows frequent or even continuous monitoring of the ventilation of a patient by measuring non-invasively the arterial *p*CO₂ with a precalibrated and easy to use monitoring system.

Thus, whereas the previously available systems are based on electrochemical principles and need to be frequently recalibrated and thus must include a calibration unit, the new system detects the chemical substances by optical means in a small optical sampling cell at the surface of a transcutaneous sensor by using an optical technique, preferably a modulation spectroscopy technique. The sensor is precalibrated at the factory and is free of any drift.

It should be understood, however, that the sensor system, beyond the transcutaneous application, may be used for monitoring chemical substances within a large number of technical fields, including other clinical fields, chemical and food industry, bio-degradation, and for the monitoring of environmental parameters. Thus it may used in various fields of chemistry, biology, physiology, gas analysis, gas safety, monitoring of gas production and in microstructure processing, automotive, environmental, biological and food industries as well as within gas and liquid chromatography.

The sensor system of the present invention allows the operator to measure permeating gases and very small gas flows. For example, a few cubic micro-liters per minute of gas can be measured in the minute range response time, which is the case in transcutaneous blood gas monitoring where a very small gas flow is permeating out of the patient skin. The system may determine gas concentrations either applied on the medium, meaning at the interface, (e.g. on human skin with a surrounding adhesive, built-in on a bioreactor wall, on a foil to characterize its gas diffusion properties) or directly within the medium, meaning as a probe (e.g. inside the human stomach for gastric gases analysis, for bio-degradation monitoring at different depth in the bio-reactor).

Out of the all the chemicals and isotopes potentially measurable by the present system, a non-exhaustive list of the most common targeted chemicals includes CO₂, O₂, H₂O, NO₂, C₂H₄, NH₃, CO, HBr, HF, C₂H₂, H₂S, HI, CH₄, HCN, NO, SO₂, HCHO, N₂O, HCl, NO₃, and CH₃COCH₃ (acetone). In the field of medical applications CO₂, O₂, H₂O, CO, anaesthetic gases, N₂O and acetone are of particular interest.

As used herein, the term "optical sampling cell" means a body for holding a sample for analysis by optical means. It should be understood that the term "cell" relates to the any sampling chamber within the cell as well as to the body of the sampling cell.

As further used herein, the terms "sampling chamber" means a chamber within the sampling cell for receiving a sample of the chemical substance. The sampling chamber has a certain volume and may be void, or it may be filled with a porous material which allows unhindered diffusion of the sample of gas, however, which in turn reduces the volume of the sampling chamber available for the gas.

It should be understood that the propagation of the optical beam may take place within the sampling chamber as well as within the body of the optical sampling cell. It should be further understood, that such propagation is considered as free-space propagation as opposed to any waveguide propagation where the waveguide core or cladding is based on a bulk solid phase material of higher refractive index than free air or gas. Thus, whereas with the waveguide propagation the sensing is obtained through its evanescent wave, which is a non propagating wave mostly defined with an exponential decay perpendicular to the direction of propagation, the beam propagation of the present invention is meant to perform the sensing through its propagating wave within the direction of propagation.

The term "light emitter" shall mean any means which provides, generates, shapes and transmits the light beam into the sampling cell. Thus, light emitter shall comprise light source, light source controller, light transmitter, e.g. a fibre, lenses, collimators and beam splitters.

As used herein, the term "free-space propagation" means that the light propagates within the gaseous phase of the sampling chamber, within any porous material of the sampling chamber or within the body of sampling cell, however not restricted by any kind of solid state waveguide as otherwise described in the prior art, e.g. in WO03/023374.

The term "light beam optical path" should be understood as the propagation path of the light beam within the optical sampling cell. The propagation may take place within the gaseous phase of the sampling chamber, within any porous material thereof as well as within the body of the optical sampling cell. It should, however, be understood that interaction with the gaseous sample takes place only within the gaseous phase of the sampling chamber and not within the body of the sampling cell, so that interaction between the light beam and the chemical substance may appear only along part of the light beam path.

The term "light receiver" shall mean any means which receives, transmits and transforms the light beam from the sampling cell. Thus, light receiver shall comprise light detector, light transmitter (e.g. a fibre), transformer, (e.g. filter, analogue and/or digital electronics, microcontroller, signal analysis/fitting, normalization process, calibration algorithm, security controller, communication controller) and amplifier.

According to the invention the volume of the sampling chamber is at most 20 mm³.

Fetzer, in "Tunable diode laser absorption spectroscopy in coiled hollow optical waveguides", Applied Optics, June 2002, vol. 41, no. 18, discloses a sensing system have a flow rate of 60 cm³ per minute. Such system has a short response time. The system is based on a hollow optical fibre of Ø=1 mm and a length of approx. 4 m corresponding to a sampling chamber volume of approx. 4cm³. According to the disclosure of Fetzer, the sampling volume of such system may be reduced to 50 mm³ (Ø=0.25mm and length 1 m), however, not beyond that figure. In this way the system may be adapted for exhaled breath analysis, but not for transcutaneous blood gas monitoring, which require volumes in the mm³-range and transport related to gas diffusion only. The present invention solves this problem by reducing the sampling volume below 20mm³, allowing gas concentration equilibrium to be reached in a short response time. For example, with the application of oxygenation monitoring of transplanted skin, local sensing of blood gases permeating through the skin and indicating the success of the transplantation is required. In this case, the blood gases flow through the skin is limited by skin diffusion and any sampling volume larger than 20mm³ would increase the response time to an unacceptable level for this application.

According to a preferred embodiment of the invention the volume of the sampling chamber is at most 5 mm³.

A key application of the sensor system of the present invention is the continuous blood gas monitoring for neonates or adults. Such ventilation determination requires a short response time of less than 1 minute to avoid any cerebral damage (hypoxeamia or hyperoxeamia), e.g. in case of cardio-pulmonary disorder. The present invention solves this problem by the definition of a sampling volume smaller than 5mm³ allowing the gas concentration equilibrium to be reached in the required short response time. Such smaller sampling volume may be obtained in at least two ways, either by adapting the sampling chamber to the shape of the light beam emitted so that the sampling cell do not perturb the light beam or by adapting the sampling cell so that the light beam is guided but still propagating in free-space.

According to a further preferred embodiment of the invention the volume of the sampling chamber is at most 0.5 mm³.

Among the applications of the sensor system of the present invention, biodegradation monitoring and control requires an ultra-small sampling volume, below or in the order of 0.5mm³. Such low sampling volume allows real-time resolution and in turn continuous control of the bioreactor substrate and oxygen supply for optimal degradation speed.

For example, Grima, in "A New Test Method for Determining Biodegradation of Plastic Material Under Controlled Aerobic Conditions in a Soil-Simulation Solid Environment", Journal of Polymers and the Environment, vol. 9, no. 1, January 2002 discloses the degradation of cellulose in soil providing 10mm³/min (20 µg/min) of CO₂ from a 400cm³ substrate volume. Assuming a cubic substrate of a total surface of 326cm², the resulting gas permeation rate is 0.03mm³/(min cm²). Further, at a sensing area of 15cm² attached to the cellulose bioreactor, a sampling volume of 0.5mm³ corresponds to a response time of the present sensor system of less than 60sec, which is reasonable for biodegradation monitoring and control. The expensive mass spectrometer or liquid chromatography usually applied for degradation monitoring does not resolve such short response time.

With the present invention the design of the sampling chamber (or part of it) is preferably adapted to the light beam path and the light beam cross-section along the path, in order to optimize the sampling chamber volume (and consequently the system response-time) without perturbing the light beam. By "without perturbing the light beam" it is meant that the light beam perturbation generating parasitic noise (interferometric noise) stays negligible compared to the required sensor system performance, e.g. resolution, stability, and accuracy. For example, Gaussian beam equations can be used for such adaptation.

In a preferred embodiment of the invention, the internal surface of the sampling chamber is coated, preferably with a chemically and/or optically active coating. Chemical coatings may catalyse certain chemical reactions which may convert the chemical substance into another chemical substance, which may be more easily detectable than the first chemical substance. Optical coatings may help for either guiding or shaping the light beam, or for reducing interferometric/parasitic noise.

In a preferred embodiment of the invention the sensor system includes a guiding structure in which the light beam propagates.

As used herein, the term "guiding structure" means a hollow cavity acting as a mean to guide the free-space propagation of the light beam within and optionally beyond the sampling cell in order to limit the extension of the light beam and potentially to extend the interaction length between the light beam and the chemical substance. By opposition the waveguide disclosed in WO03/023374 is based on a core or cladding of bulk solid phase material where most of the guided light propagates within the solid material and not in gaseous phase, i.e. free-space, as meant in the guiding structure.

It should be understood that according to the invention the guiding structure may extend beyond the sampling cell. Thus the guiding structure may be extended in the direction of the light emitter, i.e. that the guiding structure may guide the light beam before or at the entrance of the sampling cell. Likewise the guiding structure may be extended in the direction of the light receiver, i.e. that the guiding structure may guide the light beam at or beyond the exit of the sampling cell. Thus the guiding structure may be connected, e.g. by gluing or splicing, to the light emitter and/or the light receiver.

The guiding structure may be any tubular device of any cross-section, whether circular, rectangular or elliptical which shows at least partially reflecting internal surface. The term "partially reflecting" shall mean that the guiding structure is so adapted as to allow the light beam to propagate in such a way, that less than 100% of the light intensity is lost during the propagation along the guiding structure. The partial reflectivity of the guiding structure is typically provided by having a smooth surface. By "smooth surface" is to be understood that any surface structure, including any surface porosity, is significantly smaller than the wavelength of light, so that little or no scattering of the light beam occurs relating to such surface structure or porosity.

On the other hand it should be understood that the guiding structure may in fact be porous in order to allow the chemical substance to diffuse across the surface of the guiding structure. Thus, a typical implementation uses a miniature gold tube with polished internal surface achieving long interaction length, which improves the system sensitivity as well as reducing the sampling volume to provide a short response time. Preferably, the guiding structure is selected from the group of tubes (capillaries), hollow fibres, multi-hole fibres and photonic band gap devices. It should be understood, however, that any porosity of the guiding structure is on the molecular level, i.e. several orders of magnitude smaller than the wavelength of the light with sensor system.

In a particular embodiment, hollow core optical fibres and hollow core photonic crystal fibres (hollow core PCF) wherein up to or even more than 95% of the light is travelling in the hollow core, are used for free-space propagation along the guiding structure. The sensing performed by such fibre is obtained by propagating waves.

Alternatively, the guiding structure is non-porous to the gaseous chemical substance.

In most cases the guiding structure is chemically inert, e.g. as the above gold tube. In a preferred embodiment of the invention, however, it may be coated with a chemically and/or optically active coating. Chemical coatings may catalyse certain chemical reactions which may convert the chemical substance into another chemical substance, which may be more easily detectable than the first chemical substance. Optical coatings may interact with the chemical substance to create a detectable output signal.

In a preferred embodiment of the invention the guiding structure receives the gaseous sample. Thus, as the guiding structure is at least partly arranged within the sampling cell, it extends across the sampling chamber thereof, the guiding structure may be the part of the sample cell which receives the gaseous sample. Such arrangement of the guiding structure further reduces the volume of the sample chamber and the response time required for a proper detection of the chemical substance.

In one embodiment of the invention the guiding structure is straight. Such straight guiding structure provides the smallest sampling chamber volume and may be applicable with sensor systems of high sensitivity and easily detectable chemical substances.

In another embodiment of the invention the guiding structure is folded, U-shaped or spiral-shaped. With such guiding structures the light beam optical path is extended, while still keeping the volume of the sampling chamber and the overall sample cell volume at a low level. The extended light beam optical path, however, improves the system sensitivity, and may as such be applied with less detectable chemical substances.

In a preferred embodiment of the invention the light beam propagates by single monomodal propagation.

By "monomodal propagation "is meant a light beam propagation with no interaction or interference between individual optical modes. Thus "monomodal propagation" covers that only one optical mode propagates along the system, or, in case a plurality optical modes propagates, each mode propagate without interfering with any other propagating mode. For example, the sampling chamber surrounding the light beam should keep the propagation monomodal and not perturb the light beam at point where the free-space propagating mode is converted in several modes as otherwise interference and parasitic noise occur.

By "Single propagation" is meant a uniquely forward propagation of the light beam where reflection, refraction, diffraction or scattering of the light beam do not occur or do not interfere with the light beam itself.

Typically, etalon fringes, which are considered as the main contributor to parasitic noise, originate from a partial back reflection folding the beam on itself and interfering with it. With the present invention the entire system and in particularly the sampling cell are adapted to the beam shape, its cross-section and its optical path in order to fulfil the single monomodal propagation for the high performance sensor system. For example, the shape of the sampling chamber can be fabricated according to the Gaussian beam shaped by light emitter, so that negligible perturbation occurs.

The single monomodal propagation of this preferred embodiment of the invention ensures the highest possible reduction of the any kind of parasitic noise, originating from refractive index discontinuities, intermodal interferences, beam propagation perturbation, beam partial scattering, etc. Such noise reduction may be achieved if the beam stays monomodal and propagates one single time from, and along, the light emitter to, and along, the light receiver.

In a preferred embodiment of the invention the light emitter comprises a collimator. By means of the collimator, the light beam may be limited and defined in terms of direction and angular divergence. In this way parasitic noise may be reduced.

For the best possible light beam propagation, and in order to further reduce interferometric noise, at least one of the light emitter and the light receiver preferably comprises a beam shaper. By "beam shaper" it is meant any device to modify the light beam wavefront, preferably a collimator, a lens, a gradded index lens (GRIN, Selfoc) or diffractive grating. Preferably the light emitter comprises a beam shaper.

One embodiment of the present invention uses an optical waveguide on at least one of the light emitter and/or the light receiver. In a preferred embodiment an optical waveguide is used on the light emitter. In another preferred embodiment of the invention an optical waveguide is used on both of the light emitter and the light receiver. Preferably the optical waveguide is a fibre or a lensed-fibre, which latter also fulfils the collimator or beam shaper function. Fibres and lensed-fibres ensure a proper transmission with a minimum of loss and noise from the emitter to the sampling cell and from the sampling cell to the receiver.

One embodiment of the present invention uses a lensed-fibre encompassing the collimator (lens) within the fibre tip (the lensed-fibre may be considered as a drop-like ball (or particular shape) of glass formed, glued or soldered at the fibre tip). The lensed-fibre output wavefront may be customized from strongly focusing (typically in the range of a few microns for beam-waist-diameter and a few microns for distance-to-beam-waist) to almost collimating (typically less than one hundred microns for beam-waist-diameter and several millimeters for distance-to-beam-waist). Diverging beam is also possible. In combination with a sampling cell closely surrounding the light beam the resulting sensor system reaches short response time required in many critical application fields.

In a preferred embodiment of the invention the light emitter includes a beam splitter. By means of the beam splitter the light beam is splitted into a sampling cell light beam and a reference light beam. It should be understood that the sampling cell light beam is passed across the sampling chamber, whereas the reference light beam is not to be passed across the sampling chamber. Instead, the reference light beam propagates to the light receiver, e.g. in an additional waveguide, or across the sampling cell, however, without interacting the sensed chemical substance. It is preferred to use the reference light beam for comparison to the sampling cell beam for better system performance, e.g. common-mode rejection.

In a preferred embodiment of the invention the light emitter is wavelength-modulated or frequency-modulated. Further, the light receiver may be adapted for fundamental and/or harmonic detection.

In a preferred embodiment of the invention, and combined or not with the above wavelength- or frequency-modulated light, the wavelength (or frequency) may be swept over the wavelengths (or frequencies) of an appropriate absorption peak of the chemical substance. The resulting signal, corresponding to the signature of the absorption peak, may be compared to a 100% transmission and/ or a reference signal to produce the chemical substance signal. Without wavelength-modulation (or frequency-modulation) the direct absorption gas signal is generated, combined with wavelength-modulation (or frequency-modulation) the harmonic (also called derivative) signature of the absorption peak is generated.

With alternative embodiments of the invention, the light receiver may be adapted for fundamental and/or harmonic detection. With fundamental and harmonic detection by the light receiver the first, second or higher order derivative of the absorbance spectrum may be detected. In case the demodulation is performed at a frequency equal to the wavelength modulation frequency, the first derivative of the absorption spectrum may be observed, referred to as fundamental detection (or first harmonic detection). In case the demodulation is performed at a frequency twice the wavelength modulation frequency, the second derivative of the absorption spectrum may be observed, referred to as the second harmonic detection. Using a similar technique, higher harmonics may be detected as well. A combination of different harmonics detection may also be applied for better system performance.

Wavelength- or frequency modulated spectroscopy and fundamental and harmonic detection allow for a highly efficient noise reduction and a high sensitivity of the sensor system.

In a preferred embodiment the sensor system applies wavelength modulation of a laser diode by modulating its current or temperature and performs narrow bandwidth detection at the fundamental or/and harmonic(s) of the modulation frequency.

Thus, in a preferred embodiment of the present invention wavelength modulation is used to minimize noise contamination of the signal delivered by modulation techniques. From the state of the art it is known that signal-to-noise ratio in high sensitivity wavelength modulation spectroscopy is limited by the so-called "interferometric noise" originating from light interference between two or a plurality of partially reflecting interfaces or intermodal interference. For instance, non-adiabatic transitions of the refractive index along the light propagation, provides partially reflecting interfaces. Discrete elements based sensor systems suffer from multiple air-glass interfaces (fibre-air, air-collimator lens, etc) generating interferometric noise.

In order to reach the high sensitivity with modulated spectroscopy, interferometric noise should be reduced. In a preferred embodiment this may be done by the use of a single mode light emitter such as a DFB laser diode or a VCSEL or any kind of suitable laser providing a narrow spectral emission (related to the gas absorption peak width), coupled to a single-mode fibre, itself coupled as a single mode in free-space and propagating in the sampling cell. The light beam, in turn, may be described by a Gaussian beam (Hermite - Gaussian , Laguerre - Gaussian or Bessel beams) which can be easily generated and further collected by a lensed fibre.

Further, statistical treatment is used to eliminate the residual interferometric noise. Statistical treatment may be applied to the signal after intentionally perturbing or scrambling some parameter affecting residual interference, e.g. by means of a thermomechanical actuator. Spectral averaging contributes to interference noise rejection.

In a preferred embodiment of the invention a calibration-free operation of the sensor system may be obtained by comparing, in the light receiver, the chemical substance signal to the direct transmission of the reference signal or to the sampling cell signal acquired outside the sensed chemical substance absorption spectra. In the case of wavelength-modulation or frequency-modulation, calibration-free operation may also be obtained by comparing the harmonic signal to the sampling cell direct transmission, to the direct transmission of the reference signal, or to the harmonic signal of the reference signal. Any combination of the above comparisons may also be applied and is hereby referred as "normalization process".

With calibration-free operation of the sensor system, the signal reflecting the chemical substance may be determined by an in-situ calibrated process which is completely free of any other calibration process. In particular, the determination is free of any calibrations process step which should otherwise be performed as separate steps before or after the sample determination.

In a preferred embodiment of the invention the shape of the light beam optical path is modified by refraction, reflection, diffraction or scattering within the sampling cell, within the sampling chamber or within the guiding structure.

By such modification of the light beam optical path, the path length is extended in order for the light beam-chemical substance interaction to be increased. With each of the phenomena of refraction, reflection, diffraction and scattering the light beam optical path is deviated from straight propagation and the path length is consequently extended.

It should be understood that according to the invention the above light beam path modification in terms of refraction, reflection, diffraction or scattering is performed in such a way that it does not introduce any significant interferometric noise. Thus the modification is done in such a way that the refracted, reflected, diffracted or scattered light beam does not interfere with it self, and that no additional interfering light beam modes are introduced with the refraction, reflection, diffraction or scattering.

For example, the light beam from the light emitter, propagating in the sampling cell, may be split by the use of a beam-splitter cube generating a reference signal and a sampling cell signal through refraction and partial reflection. Then the optical path in the sampling cell can be folded by the use of a mirror (flat or concave with total reflection) so that the gas - light beam interaction is doubled while the sampling cell is kept compact. The folded light beam must be tilted to avoid back-reflection interference. The light receiver may be placed next to the light emitter.

The sampling cell of the sensor system according to the invention preferably comprises a membrane. The membrane may be arranged with the sampling cell adjacent to the sample chamber.

With the membrane any unwanted interaction between the medium and the light beam may be reduced. Indeed, many applications require separating the sensed chemical substance from the rest of the medium. Thus, for sensing of a gaseous chemical substance, the membrane prevents liquids or solids from the medium from interacting with the light beam, while allowing the gaseous chemical substance to diffuse across the membrane into the sampling chamber.

It should be understood that the membrane may have varying properties relating to the diffusion of the chemical substance or the propagation of the light beam. Thus, the membrane may be coated and/or may have bulk properties, i.e. the membrane material itself, with a chemical or optical activity. A chemical activity may allow the conversion of the chemical substance into another substance, which may be easier to detect. In a preferred embodiment of the invention the membrane comprises enzymes which may convert the chemical substance into another substance which may be easier to detect. Thus, the membrane may comprise glucose oxidase, which converts glucose into hydrogen peroxide. An optical coating of the membrane may interact with the light beam for supporting the sampling cell functionalities.

The membrane may be chemically inert or it may be reactive as described above. Preferably it should be selective. For this purpose, it has a selected porosity and/or chemical structure.

The membrane may further be based on a sandwich structure to fulfil several requirements such as separation, prevention of fouling, mechanical rigidity and ease of assembly.

The membrane may further have a controlled surface roughness or a controlled surface structure. Thus, the membrane may be adapted to shape the part of the sampling cell receiving the gas sample in such a way that a maximum amount of sample may be received while keeping the volume of this part as small as possible. It should be understood that the sampling cell should still allow a proper diffusion of the chemical substance within the sampling cell. This may be achieved by a sampling cell construction according to which the part of the sampling cell receiving the sample is defined by the membrane surface roughness or the membrane surface structure.
Thus the membrane may be arranged adjacent to the sampling cell wall so that the only space for diffusion of the chemical substance between the membrane and the sampling cell wall is the space left by the membrane surface roughness or structure. The combination of the membrane roughness/structure with the sampling cell wall roughness/structure also applies to allow proper gas diffusion while keeping the volume of the sampling cell small.

The membrane may be fixed with or removable from the sampling cell, or the membrane may be integrated within part of the sampling cell to form an exchangeable/disposable part of the system.

In a preferred embodiment of the invention the membrane and at least part of the sampling cell are integrated into a sampling cell assembly. According to this embodiment the sampling cell assembly may be exchangeable or disposable.

With an exchangeable sampling cell assembly the assembly may be adapted for various measurement conditions like sampling volume (e.g. for adapted measurement on adults and enfant), temperature and humidity.

With a disposable sampling cell assembly the assembly may be changed with every single measurement, in order to minimize inter-sample contamination.

With another preferred embodiment of the sensor system according to invention the system comprises a medium pipe crossing the light beam.

The term "medium pipe" shall mean a flow structure for continuously transporting the medium across the light beam for interaction therewith. Such medium pipe is especially applicable for the continuous monitoring of chemical substances, e.g. in gas production environments.

It should be understood that the medium pipe may be an integrated part of the sampling cell or it may be arranged adjacent to the sampling cell in such a way that the chemical substance may continuously be monitored by the sensor system when flowing in the medium pipe.

In a preferred embodiment the sampling cell has a sampling area for sampling the chemical substance.

The terms "sampling area" shall mean an area of the sampling cell devoted to sample collection. Preferably the area of the sampling area may be relatively large. Thus, typically the sampling area may be a high surface structure, defining a small volume for collecting the sample, e.g. upon diffusion thereof across a membrane, and in turn allowing the gas to diffuse to the sampling chamber. The volume defined by the sampling area should be minimized in order to reduce the response time of the sensor system. In one preferred embodiment of the invention the sampling area is defined by a membrane arranged adjacent to the sampling cell wall so that the only space for diffusion of the chemical substance between the membrane and the sampling cell wall is the space left by the membrane surface roughness or structure and the sampling cell wall roughness or structure. Likewise, the surface of the sampling area may have a controlled surface roughness or a controlled surface structure defining such very small volume available for diffusion of the sample of the chemical substance.

The arrangement of the light beam optical path relative to the sampling area may be accomplished in at least two different ways.

Thus, the light beam optical path may be situated adjacent to the sampling area so that light beam optical path is in direct contact with the sampling area. In this way the gaseous sample diffuses direct from the sampling area to the light beam optical path.

Alternatively, the light beam optical path may be embedded in the optical sampling cell. In such case the sampling cell may further comprise at least one duct between the sampling area and the light beam optical path for diffusion of the gaseous chemical substance from the sampling area to the light beam optical path.

The light emitter and the light receiver of the sensor system according to the invention are preferably integrated with the optical sampling cell. Thus the light emitter may be integrated with the optical sampling cell. Likewise, the light receiver may be integrated with the sampling cell. Preferably, both of the light emitter and the light receiver are integrated with the sampling cell.

The term "integrated with the optical sampling cell" shall mean that the light emitter and/or the receiver may be held by the sampling cell, e.g. glued or otherwise attached thereto or assembled therewith, or it may be fully integrated therewith, i.e. manufactured as on one single structure, e.g. on one single wafer board.

It should be understood, that according to the invention, the sampling cell may integrate up to the entire sensor system including any opto-electronic detection circuit, controller, the light source, any beam splitter including any transmitters. In such case, the sampling cell may be a semi-conductor, like silicon or III-V components, taking advantages of the micro-electronic fabrication processes.

Preferably the sensor system according to the invention is temperature controlled. Thus the sensor system may comprise a heating system, such as a heating coil, a Peltier element and/or a heating electronic device. Likewise, the sensor system may comprise a temperature sensing system, such as thermistors and/or electronic chip sensors.

In a number of preferred embodiments of the invention the sensor system may be adapted to multiple detection. "Multiple detection" covers the detection of additional chemical species or isotopes, the simultaneous detection of the same chemical species in a plurality of samples as well as the simultaneously detection of different chemical substances from different samples.

Thus, the sensor system may further comprise at least one additional optical sampling cell, which in turn may be arranged in series or in parallel with the optical sampling cell. Preferably the optical sampling cell and the at least one additional sampling cell are integrated in a single sampling cell body.

For the detection of more chemical substances or for the detection of the chemical substance in more samples, the sensor system may comprise at least one additional light emitter. Likewise, the sensor system may comprise at least one additional light receiver. Preferably the sensor system may comprise an additional light emitter and an additional light receiver, which are adapted work in combination. Alternatively, the additional light receiver may work along with the light receiver, detecting e.g. different harmonics of the light signal or combined with a different wavelength-modulation or frequency-modulation.

In a further alternative embodiment of the invention the light emitter is a tunable wavelength light emitter for detection of multiple chemical substances or multiple isotopes.

According to another aspect of the invention a sampling cell assembly for use with a sensor system according to the first aspect of the invention is provided, said sampling cell comprising an optical sampling cell holding a sampling chamber for receiving a sample comprising the chemical substance, and a membrane adjacent to the sampling chamber.

As described above the sampling cell assembly may be disposable to minimize inter-sample contamination.

In the following different embodiments of the invention are described with reference to the accompanying drawings in which:
Fig. 1 shows a schematic of the sensor system according to the invention.
Fig. 2 shows a schematic of the sensor system with a fibre-based light emitter and receiver and a membrane.
Fig.3 shows a schematic of the sensor system with a lensed-fibre light emitter and light receiver and a reference light beam.
Fig. 4a and 4b show a schematic of the light beam optical path including a deviated and/or split light beam path.
Fig. 5 shows a schematic of the sensor system with a guiding structure.
Fig. 6 shows a schematic of the sensor system with an embedded guiding structure and ducts.
Fig. 7 shows a schematic of the sensor system with a spiral-shaped guiding structure.
Fig. 8 shows a schematic of the sensor system with a folded light beam optical path including micro-mirrors.
Fig. 9 shows a schematic of a sensor system with a medium pipe.
Fig. 10 shows a schematic of the sensor system with a sampling area structure.
Fig. 11 shows a schematic of the sensor system with additional sampling cells connected in series and parallel.
Fig. 12 shows a schematic of the sensor system with multiple light emitters.

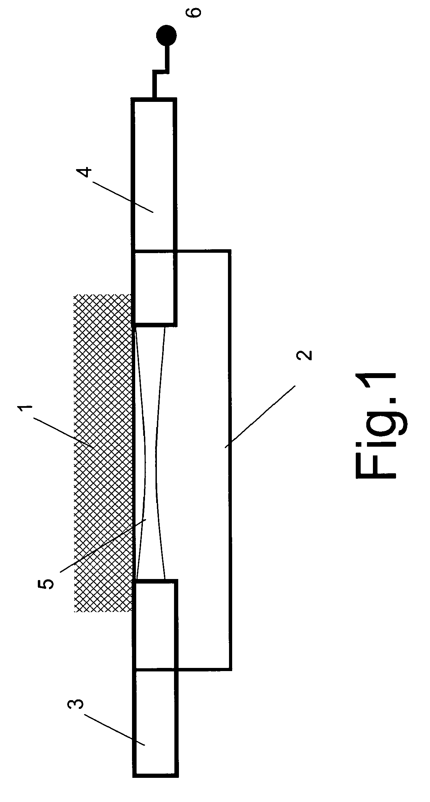

The sensor system according to the invention is shown in Fig. 1. The sensor system is adapted for detection of a chemical substance in a medium (1), and comprises a sampling cell (2), a light emitter (3) and a light receiver (4) arranged to produce and detect a light beam (5).

The light emitter may be any device based on a light source, like lasers, laser diodes and light emitting diodes, which light is coupled into the sampling cell, producing the light beam. Reciprocally, the light receiver may be any device like an opto-electronic circuit for detecting light from the sampling cell to produce an output signal (6) reflecting the sensed chemical substance concentration in the medium.

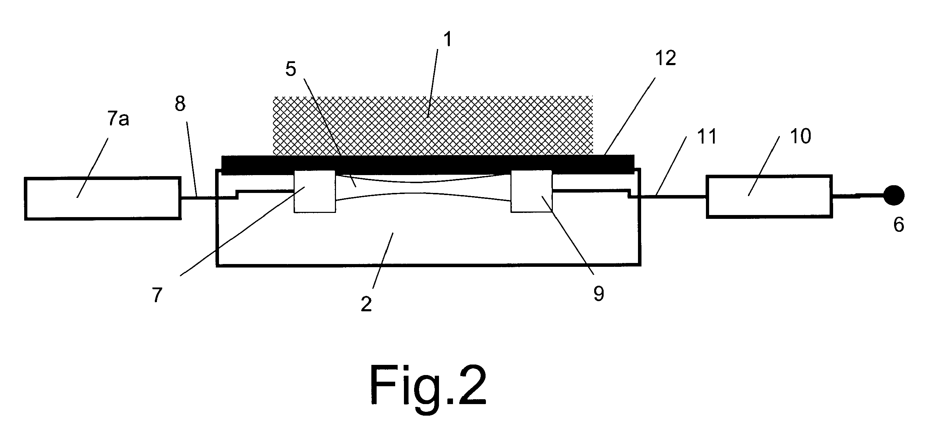

The sensor system shown in Fig. 2 shows that the light source (7) of the light emitter may be held by the sampling cell (2) and controlled through a transmitter (8), e.g. by a laser controller (7a) in terms of current, power and/or wavelength, so that the intensity of the emitted light beam (5) is processed.

The sensor system shown in Fig. 2 also shows that the detection circuit of the light receiver being connected to the sampling cell. The measurement signal of the detector (9) is delivered to the electronic detection circuit (10) through the transmitter (11) like an optical lensed fibre, a simple electric wire or a wireless transmitter.

The sensor system shown in Fig. 2 also includes a membrane (12) separating the medium (1) and the sampling cell (2). The membrane prevents part of the medium to interact within light beam.

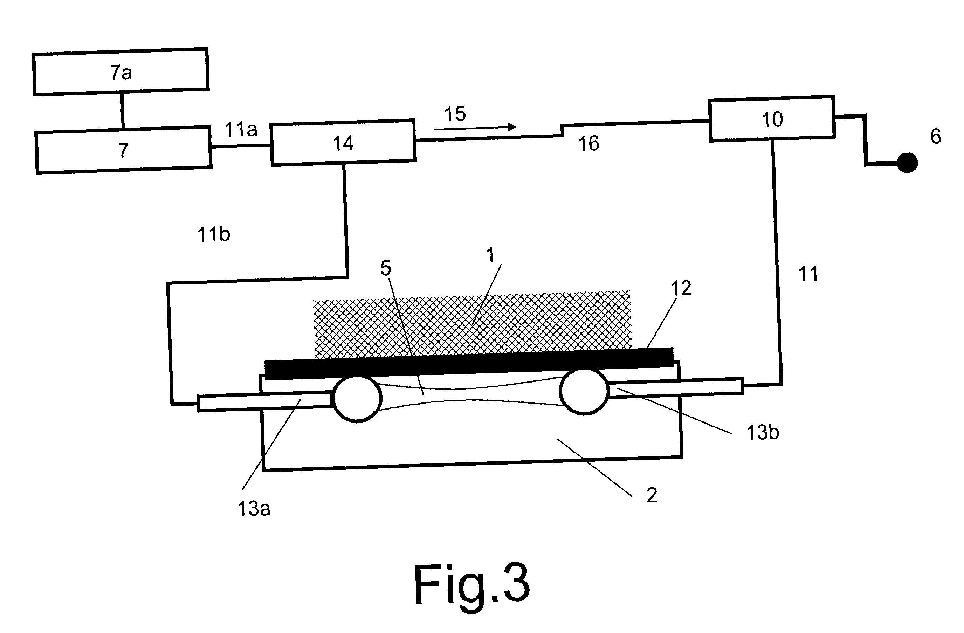

With the sensor system variant shown in Fig. 3 both of the light emitter and the light receiver are based on lensed-fibres (13a, 13b), for coupling light from the light source into the sampling cell, for producing the light beam, and for detecting the light beam transmitted to the electronic detection circuit. It should be understood that the light source and the electronic detection circuit may be located close to or even integrated within the sampling cell or in a remote position from the sensing location. Remote sensing is typically the case for transcutaneous blood gas monitoring when the sensing takes place on the patient skin and the light source and/or output signal monitor is/are located on the patient bedside. The lensed-fibre may in turn be replaced by any fibre-like waveguide, with or without polarization maintaining properties, which fibre-like waveguide tip is modified as or assembled to a lens or any device fulfilling similar purpose in order to change the output beam wavefront, e.g. divergence, collimation or focusing.

The sensor system shown in Fig. 3 also shows a beam splitter (14) like a fibre optical coupler or free-space beam splitting prism to produce a reference signal (15) for differential detection and/or balanced detection. The reference signal may be given by the light emitter, or sampled from the light beam before or along sensed chemical substance interaction, and transmitted to the light receiver by a transmitter (16) like an optical fibre, a waveguide, an electric wire or even a wireless system.

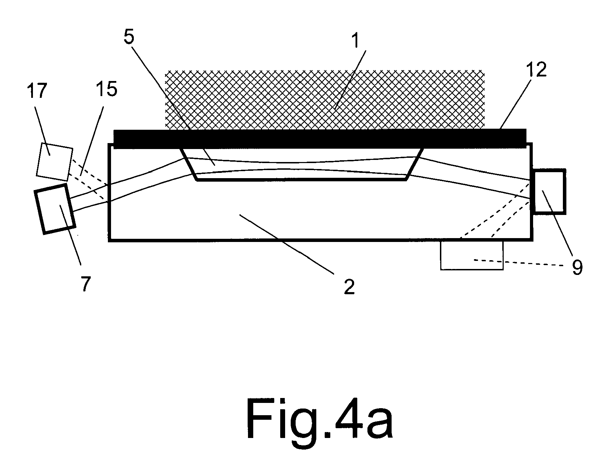

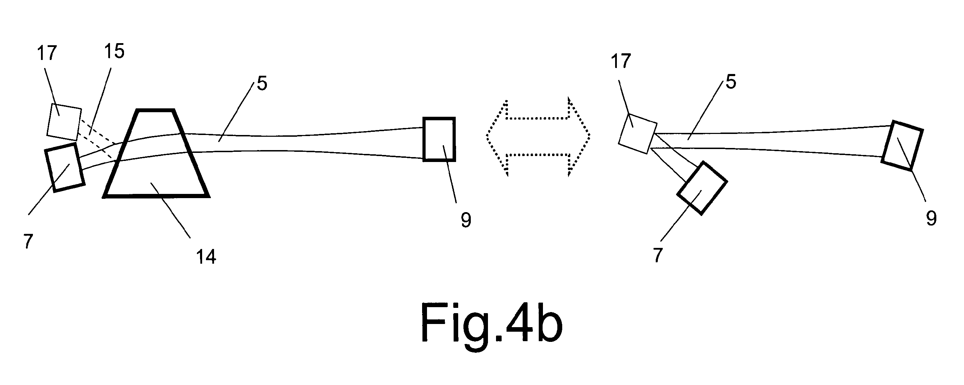

The sensor system shown in Fig. 4a uses the advantage of deviating the light beam by refraction, reflection, diffraction or any combination thereof, thereby extending the light beam optical path. In this case a beam splitter generates a reference signal (15) which may be detected by an additional detector (17). With this figure, and with Figure 4b, solid lines represent refraction, whereas dashed lines represent refraction and reflection.

Figure 4b shows how a prism (14), deviating and splitting the light beam, may be reduced to the detector (17) used as partial reflector and providing simultaneously a reference signal and the deviation of the light beam. This may be considered as the integration of the detector in the beam splitter and vice-versa.

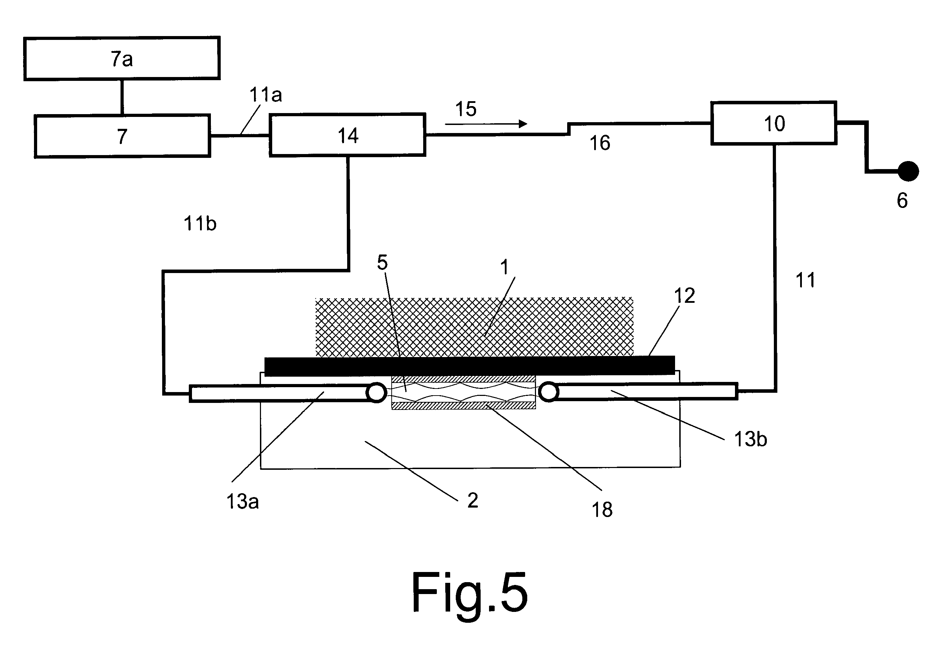

Fig. 5 shows the sensor system including a guiding structure (18) which is a hollow cavity acting as a mean to guide the light beam. With the guiding structure the extension of the light beam is limited, and the interaction length between the light beam and the chemical substance may be extended.
The internal surface of the guiding structure may have particular absorption, reflection or scattering properties given by the microstructure itself or any additional surface treatment, such as a reflecting coating.

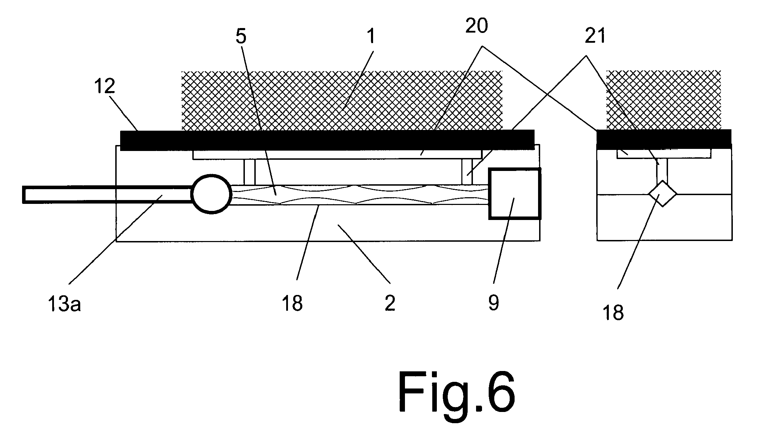

Fig. 6 shows the sensor system in which the guiding structure is embedded in the sampling cell and connected to the sampling area (20) by one or several duct(s) (21). The purpose of the duct(s) is to let the chemical substance diffuse or flow, from the collecting area, along the said duct(s), to the sampling chamber for sensing interaction.

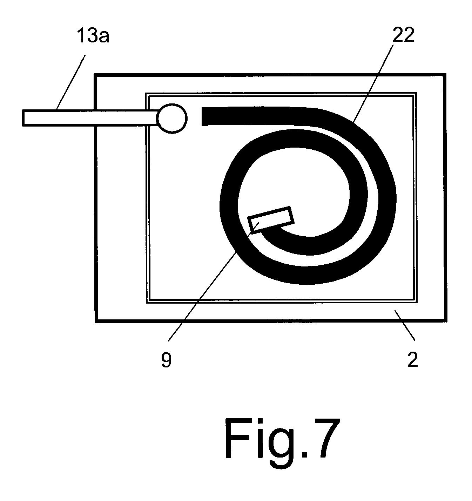

Fig. 7 shows a spiral-shaped guiding structure (22). The light beam path may have a wide range of shapes, in order to increase of the interaction length between the light beam and the chemical substance while keeping the sensor system compact. Typical shapes include straight shapes, U-shapes or spiral-shapes, which are preferably located about the top surface, i.e. close to the medium and the sampling area.

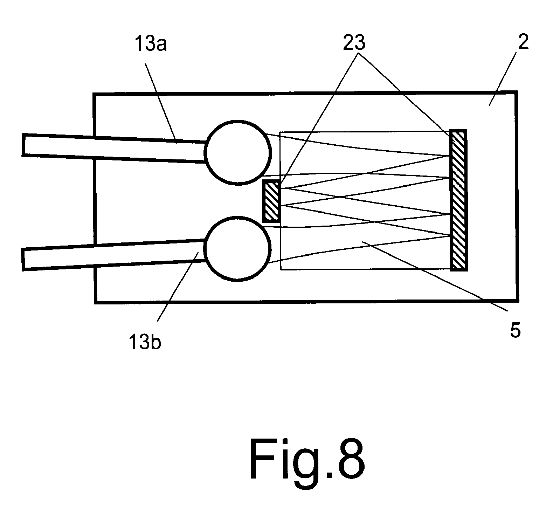

The Fig. 8 shows the sensor system with a folded optical light beam path, the light beam path being folded by means of one or more micro-mirrors (23) reflecting the light beam.

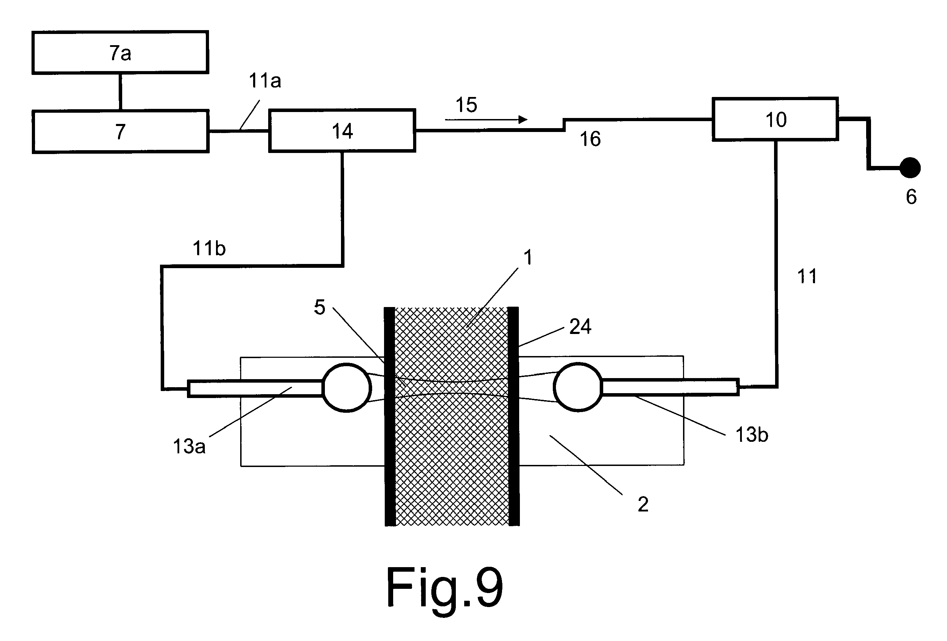

The sensor system as shown in Fig. 9 displays a medium pipe (24) which is an intermediate cavity acting as a pipe for the sensed medium and crossing the light beam. The purpose of the medium pipe is to allow the medium (1) to flow or diffuse across and not on top of the sampling cell. The medium pipe may be applied along a (micro-)fluidic system simply by connecting or integrating the medium pipe to (micro-)fluidic system pipeline.

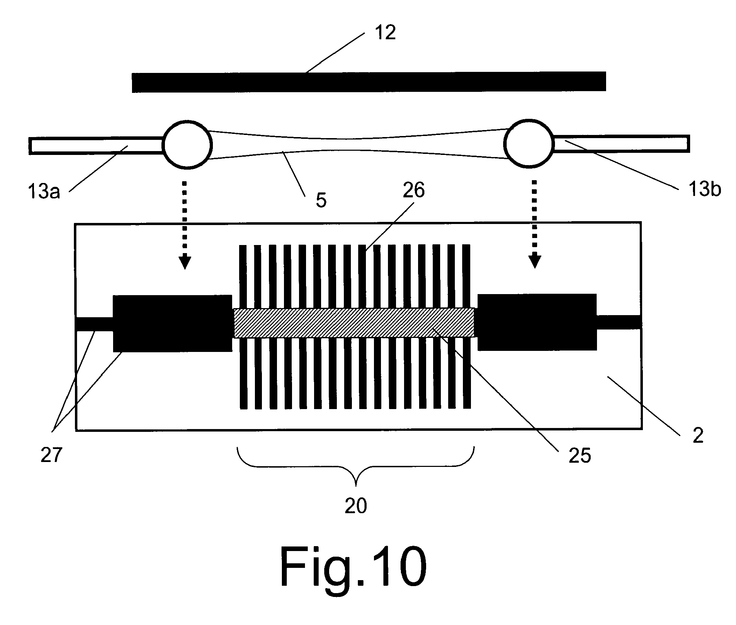

An important parameter for the sensor system of the invention is the response time. The response time in turn is related to sampling structure, i.e. to optimize the ratio of the sampling area to the sampling chamber. Fig. 10 shows the sensor system with a sampling area (20) with a sampling microstructure (26). The sampling area should be as high as possible to gather the sensed chemical substance, from a large surface towards the sampling chamber (25). The sampling area is connected directly or indirectly to the sampling chamber allowing the chemical substance to diffuse or flow towards the light beam for sensing interaction. Thus, the sampling area may be integrated within the sampling cell, it may be built as a discrete element or it may be integrated with the membrane. The microstructure may be of various cross-sections but preferably have a shallow depth so that its contribution to the total sampling volume is minimized. The conformation of the sampling area, meaning the arrangement of the microstructure may vary from the absence of micro-channels to striped, crossed or fractal conformations. Also the manufacturing process, meaning the surface roughness, may be considered as a microstructure for the sampling area. Even a porous layer may be seen as sampling area microstructure. The sampling area may be expanded to the entire sampling cell top surface and even wider over other facets and/or over adapted elements assembled within the sampling cell.

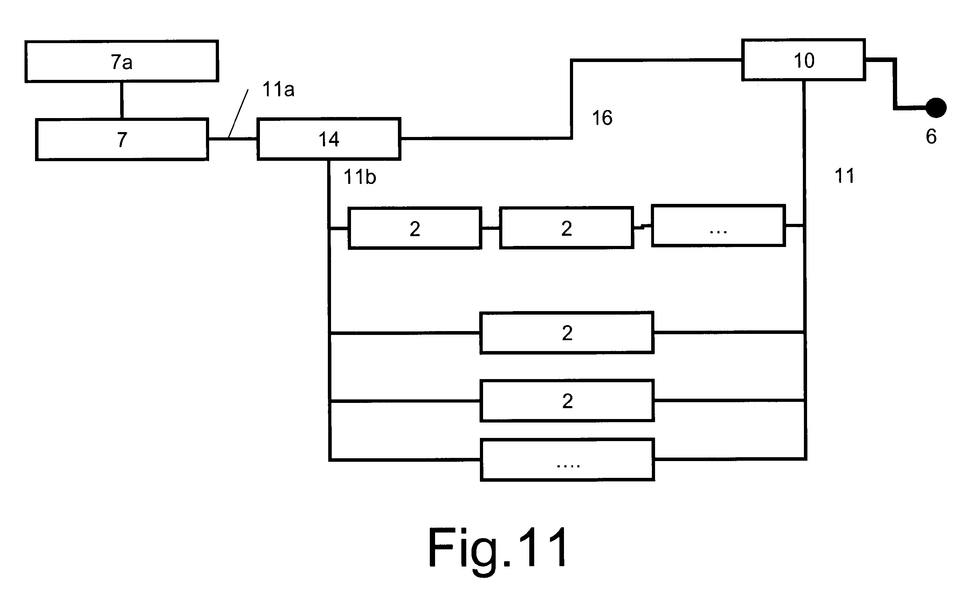

In order to sense chemicals in different locations forming a multi-point optical sensor system as schematically described in Fig.11, several sampling cells may be combined in series and/or in parallel.

Expanding the scale of integration, the series and/or parallel architectures may be integrated or assembled within a single (or a reduced number of) sampling cell body (bodies) to form a compact sensor system.

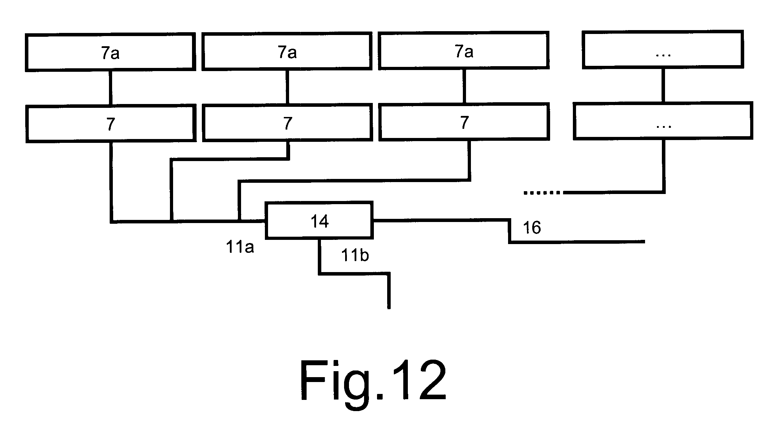

In order to sense different chemicals or isotopes, one or multiple light sources, tunable or with fixed wavelength, may be applied forming a multiple light source sensor system as schematically shown in Fig 12.

According to the targeted application, all the above described embodiments may be combined for optimal sensing.

The following describes an embodiment of the invention in which the sensor system has a sensor-head encompassing the sampling cell and remotely connected via a cable from an instrument. The instrument encompasses the laser light source, the laser controller, the beam-splitter of the light emitter, and the reference detection optoelectronics, the signal treatment electronics of the light receiver and also the power supply and the output signal display. It is applied in transcutaneous blood gas monitoring where the patient skin is heated for better correlation between arterial partial pressure and transcutaneous partial pressure (Severinghaus correction is applied). The gas monitored in this embodiment is carbon dioxide.

The laser source (20mW) emits wavelength modulated light of 1572nm or 2004nm according to CO₂ absorption peaks. The laser is wavelength-modulated at 500Hz by its driving current. The light beam is injected into a standard telecom optical fibre (SMF-28). The beam splitter provides a reference signal (10%) and a measurement signal (90%), which latter is guided along the 2m-long cable into the sensor-head. The sensor-head is based on optical sampling cell (<5mm³ sampling volume) allowing both the collection of the gases permeating from the patient skin (∅20mm sensing area) and the interaction between light and the sensed gas (physical principle of light absorption over a central channel of Ø0.4mm x 15mm). At the end of the optical fibre a micro-lens, i.e. a lensed-fibre, collimates the light beam in the central channel of the sampling cell, where the sensed gas is collected by diffusion through a Teflon membrane and ducts of a diameter of 0.2mm). At the end of the central channel an InGaAs photodetector measures the light intensity, which normalized modulation reflects the sensed gas concentration (0-760mmHg range given by fitting, linearization function and calibration factor set at factory). High sensitivity is obtained by wavelength modulation of the light and demodulation after the photodetector, comparison to the reference signal and harmonic detection provides a rejection of the common fluctuations and improves the system performance. High selectivity is given by the very narrow spectral width of the laser source targeting a single absorption line of the sensed gas (about 10GHz wide). The signal processing converts the light intensity signal into gas partial pressure (pCO₂ or tc-pCO₂ for transcutaneous) displayed at a 1sec rate.

Transcutaneous partial pressure measurement ranges from 0-250mmHg. A good correlation with the patient blood gas level (PaCO₂) is obtained by heating the patient skin temperature at 41-43°C (heating coil around the sampling area and metallic sampling cell for heat conduction) generating an arterialization of the under-skin vessels. Thus, by continuous monitoring, a patient cardio-pulmonary disorder can be detected with a response time shorter than 60sec allowing alerted medical staff to react before central nervous system damage consequently to lack or excess of ventilation.

## Claims

1. A sensor system for detection of a gaseous chemical substance in a medium, said sensor system comprising:
- an optical sampling cell holding a sampling chamber for receiving a sample comprising the gaseous chemical substance
- a light emitter for generating and coupling a light beam into the sampling cell for free-space propagation along a light beam optical path within the sampling cell for interaction with the gaseous sample held in the sampling chamber, and
- a light receiver to detect the light beam from the sampling cell and to produce an output signal reflecting the gaseous chemical substance of the sample,
**characterized in that**
the volume of the sampling chamber is at most 20 mm³.

2. The sensor system according to claim 1 in which the volume of the sampling chamber is at most 5 mm³.

3. The sensor system according to claim 1 in which the volume of the sampling chamber is at most 0.5 mm³.

4. The sensor system according to any of the preceding claims in which the sampling chamber fits or is adapted to the light beam cross-section along the light beam path.

5. The sensor system according to any of the preceding claims in which the internal surface of the sampling chamber is coated, preferably with a chemically and/or optically active coating.

6. The sensor system according to any of the preceding claims further comprising a guiding structure in which the light beam propagates.

7. The sensor system according to claim 6 in which the sampling cell comprises the guiding structure.

8. The sensor system according to claim 7 in which the guiding structure is coated, preferably with a chemically active coating.

9. The sensor system according to any of claims 7 and 8 in which the guiding structure receives the sample

10. The sensor system according to any of claims 7-9 in which the guiding structure is a hollow optical device, preferably selected from the group of tubes, hollow fibres, multi-hole fibres, photonic crystal fibres and photonic band gap devices.

11. The sensor system according to any of claims 7-10 in which the guiding structure is straight.

12. The sensor system according to any of claims 7-10 in which the guiding structure is U-shaped or spiral-shaped.

13. The sensor system according to any of the preceding claims in which the light beam propagates by single monomodal propagation.

14. The sensor system according to any of the preceding claims in which the light emitter comprises a collimator or a beam shaper.

15. The sensor system according to any of the preceding claims in which at least one of the light emitter and the light receiver comprises an optical waveguide, preferably a fibre or a lensed-fibre.

16. The sensor system according to any of the preceding claims further comprising at least one beam splitter splitting the light beam into a sampling cell light beam and a reference light beam.

17. The sensor system according to any of the preceding claims in which the light emitter is wavelength-modulated or frequency-modulated and the light receiver is adapted for fundamental and/or harmonic detection.

18. The sensor system according to any of the preceding claims in which a thermomechanical device intentionally perturb or scramble interferometric noise for its statistical reduction.

19. The sensor system according to any of the preceding claims in which the light receiver signal treatment applies a normalization process.

20. The sensor system according to any of the preceding claims in which the shape of the light beam optical path is modified by refraction, reflection, diffraction or scattering within the sampling cell, within the sampling chamber or within the guiding structure.

21. The sensor system according to claim 20 in which the length of light beam optical path is extended by refraction, reflection, diffraction or scattering within the sampling chamber.

22. The sensor system according to any of the preceding claims in which sampling cell comprises a membrane adjacent to the sample chamber.

23. The sensor system according to claim 22 in which the membrane is coated, preferably with a chemically or optically active coating.

24. The sensor system according to any of claims 22 and 23 in which the membrane material itself is chemically or optically active.

25. The sensor system according to any of claims 22-24 in which the membrane has a controlled surface roughness or a controlled surface structure.

26. The sensor system according to any of claims 22-25 in which the membrane and at least part of the sampling cell are integrated into a sampling cell assembly, and in which the sampling cell assembly is disposable.

27. The sensor system according to any of the preceding claims further comprising a medium pipe crossing the light beam optical path.

28. The sensor system according to any of the preceding claims in which the sampling cell has a sampling area for sampling the chemical substance.

29. The sensor system according to claim 28 in which part of the light beam optical path is situated adjacent to the sampling area.

30. The sensor system according to claim 28 in which part of the light beam optical path is embedded in the optical sampling cell and the sampling cell further comprises at least one duct between the sampling area and the light beam optical path.

31. The sensor system according to any of claims 28-30 in which the sampling area has a controlled surface roughness or a controlled surface structure.

32. The sensor system according to any of the preceding claims in which at least one of the light emitter and the light receiver are integrated with the the optical sampling cell.

33. The sensor system according to claim 32 in which the light emitter is integrated with the optical sampling cell.

34. The sensor system according to any of the preceding claims further comprising a heating system and/or a temperature sensing system.

35. The sensor system according to any of the preceding claims further comprising at least one additional optical sampling cell arranged in series or in parallel with the optical sampling cell.

36. The sensor system according to claim 35 in which the optical sampling cell and the at least one additional sampling cell are integrated in a single sampling cell body.

37. The sensor system according to any of the preceding claims further comprising at least one additional light emitter and/or one additional light receiver.

38. The sensor system according to any of the preceding claims in which the light emitter is a tunable wavelength light emitter for detection of multiple chemical substances and/or multiple isotopes.

39. The sensor system according to any of the preceding claims in which the sensor system is a transcutaneous sensor system.

40. The sensor system according to any of the preceding claims in which the gaseous chemical substance is carbon dioxide.

41. A sampling cell assembly for use with a sensor system according to any of claims 1-40, comprising:
- an optical sampling cell holding a sampling chamber for receiving a sample comprising the chemical substance, and
- a membrane adjacent to the sampling chamber.
